# EUROPEAN PATENT APPLICATION

(11) **EP 0 636 384 A1**
(43) Date of publication of application: **01.02.1995**
(21) Application number: 94201744.3
(22) Date of filing: 17.06.1994
(51) Int. Cl.: A61M 25/10, G01L 7/16

(54) **Balloon catheter with pressure indicator**

(30) Priority: 29.07.1993 NL 9301329
(71) Applicant: CORDIS EUROPA N.V., NL-9301 LJ Roden (NL)
(72) Inventor: Mulder, Martien, NL-9302 AS Roden (NL)
(74) Representative: 't Jong, Bastiaan Jacobus

(57) **Abstract**

The invention relates to a balloon catheter comprising a tube-like basic member (2) with a distal and a proximal end and at least two lumens each of which is connected to a connecting piece (5,8) by a single catheter section (4,7) at the proximal end. A balloon-shaped member (10) is attached close to the distal end of which an internal space is connected to one of the lumens through which it can be filled with fluid. A pressure gauge (9) has been received in the single catheter section (7) leading to the balloon-shaped member.

## Description

The invention relates to a balloon catheter. Such a catheter comprises a tube-like basic member with a distal and a proximal end and at least two lumens. At the proximal end each lumen is connected by a single catheter section to a connecting piece. A balloon-shaped member of which the inside is connected to one of the lumens, is attached to the distal end. Via the single catheter section connected with this lumen and the connecting piece, a fluid under pressure can be supplied to and removed from the balloon-shaped member in order to cause the balloon-shaped member to swell to a greater or lesser degree.

Balloon catheters of this type are generally known and are used for instance to dilate narrowed blood vessels. For this purpose the catheter is introduced in a patient and the balloon positioned in the area of the narrowing. By subsequently supplying fluid under pressure via the connecting piece concerned, the balloon-shaped member will expand, thus pushing the narrowing outwards.

With such a treatment it is essential that the fluid supplied to the balloon-shaped member is of the right pressure. When there is not enough pressure, dilatation will be insufficient whereas there will be a danger of trauma when the pressure is too great. The use of such a balloon catheter requires therefore great skill on the part of the attending physician.

The object of the invention is to provide a balloon catheter of the type described in the preamble which can be used to carry out the treatment with a greater chance of success. This is achieved with the catheter as characterized in claim 1. A pressure gauge can be made simple and tractable, hence not impeding the treatment. The pressure gauge will give the performing physician a clear indication of the pressure in the balloon, so that the treatment can be carried out effectively. The pressure gauge forms part of the connection line to the balloon, providing a very manageable assembly.

A particular advantageous embodiment is characterized in claim 2. The pressure acts on both piston parts, thus creating a reaction force directed at the small diameter of the piston. The force increases proportionally with the pressure. A spring working against the movement of the piston is consequently pushed in proportionally to the pressure, giving an indication of the prevailing pressure.

An advantageous further development is characterized in claim 3. The piston can be seen directly through the transparent housing, so that this in itself can function directly as indicating member together with a scale division on the wall.

With the measure as set out in claim 4 it is achieved that a magnifying effect occurs at the rounded corners allowing more accurate reading.

The invention will be explained in the following description with reference to the attached drawings.

Fig. 1 represents a view of a balloon catheter according to the invention.

Fig. 2 shows a cross-section of a pressure gauge to be used with a catheter as shown in figure 1.

Fig. 3 shows another embodiment of a pressure gauge also in cross-section.

Fig. 4 represents a cross-section of the housing of the pressure gauge of figure 3 at IV-IV in figure 3.

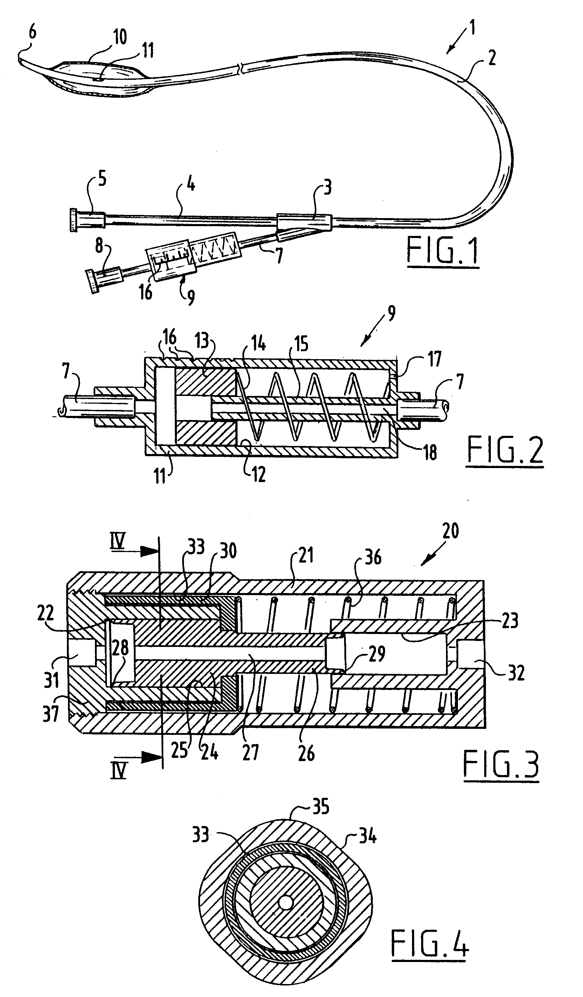

The balloon catheter 1 of fig. 1 comprises a basic member 2 with, in this example, two lumens. Close to the proximal end the basic member 2 is connected with a Y-piece 3 in which each of the two lumens is connected separately with one of the single catheter parts 4, 7. The single catheter part 4 ends in a connecting piece 5. The lumen linked to this is connected with an opening 6 at the distal end of the catheter 1 and serves for supplying contrast medium to the distal end of the catheter, so that it can be made visible on an X-ray screen during catheterization in a catheterization laboratory.

Via the above mentioned single catheter section 7, the second lumen is connected with a connecting member 8. The lumen connected to this ends in an opening 11 inside a balloon-shaped member 10 at the distal end of the catheter. By supplying fluid under pressure via the connecting member 8, the catheter section 7 and the lumen in the basic member 2 connected with it, pressure can be applied to the balloon 10, causing it to inflate.

According to the invention a pressure gauge 9 is received in the single catheter section 7 leading to the balloon-shaped member 10 which, by means of a scale division 16, gives an indication of the pressure in the line leading to the balloon 10 and hence of the pressure in the balloon 10 itself.

A cross-section of the pressure gauge 9 is shown in fig. 2. In this embodiment the pressure gauge 9 consists of an elongated housing 11 with an inlet and an outlet placed axially opposite each other to which the parts of the single catheter section 7 have been connected, for instance by means of glueing.

Inside the housing 11 a cylinder bore 12 has been formed, in which a slide piston 13 has been received. The piston 13 can also be moved along a guiding pin 15 which extends from the opposite end of the housing to inside the piston 13. Around its perimeter the piston forms a seal in the bore 12 and internally also around the guiding pin 15.

In the space with a ring-shaped cross-section to the right of the piston 13 in fig. 2, a helical compression spring 14 has been received which biases the piston 13 in the left-hand direction.

The housing 11 has been made of a transparent plastic material, on the outside of which a scale division 16 has been made.

In the guiding pin 15 a continuous channel 18 has been formed so that a continuous line is formed from the inlet to the outlet of the pressure gauge.

The pressure in the line acts on the left-hand surface of the piston 13 as seen in fig. 2. With increasing pressure, the force consequently acting on piston 13 in the right-hand direction will increase so that the piston 13 will be moved over a distance against the action of the spring 14 until a state of equilibrium has been reached. The position of the piston 13 can be related to the scale division 16 and gives in this way an indication of the pressure in the line. A bore hole 17 has been made in the housing for the escape of displaced air. This bore hole 17 is not absolutely necessary as the air in the area containing the spring is compressible, but it ensures that the displacement of the piston 13 occurs in a linear fashion with the pressure.

The pressure gauge 20 as shown in fig. 3 also comprises an elongated housing 21 with an inlet 31 and an outlet 32 on the opposite side.

Although an inlet and an outlet are mentioned repeatedly, it does not matter as regards the proper functioning of the pressure gauges described here, in which position they are received in the pressure line.

The housing 21 comprises a continuous bore hole 33. At the left-hand side in fig. 3 an insert 37 has been received in the housing. In this insert of the housing a first axial cylinder bore 22 has been formed and opposite that a second axial cylinder bore 23 has been formed in the housing, which has a smaller diameter than the first bore 22. The piston 24 is provided with sections having corresponding diameters. The left section 25 in fig. 3 fits closely in the first bore 22 and the right section 26 fits closely in the second cylinder bore 23. A channel 27 has been made through the centre of the piston, so that a continuous connection is created from the inlet, via this channel, to the outlet 32.

As the figure shows, a thin protruding edge 28 has been formed at the end of the broad section 25 of the piston and the same has been done at the narrow end 26 of the piston where also a protruding rim has been made. These rims 28 , 29 push outwards in a resilient manner, so that there is a good sealing contact with the bores 25 and 23 respectively.

A sleeve 30 is connected with the piston 24 which extends in a space with a ring-shaped cross-section between the insert 37 and the bore 33 of the housing 21. This sleeve 30 is of a contrasting colour and forms the indicating member together with a scale division on the outside of the housing 21. In the empty space inside the housing a helical compression spring 36 has been received forcing the piston 24 to the left.

The pressure in the channel between the inlet 31 and the outlet 32 acts on both sides on the piston 24. Consequently a reaction force, proportional to the magnitude of the pressure, will occur to the right. Subject to the pressure, the piston will move to the right against the force of the spring 36, until the resultant force due to the pressure is in equilibrium with the opposing force exerted by the spring 36. The position of the spring with the sleeve 30 is therefore proportional to the prevailing pressure; in other words, when the pressure increases the construction of piston 24 and sleeve 30 will take up a position more to the right.

At the outer circumference of the housing 21, itself made of a transparent plastic material, a scale division, not shown here, has been added like in the embodiment shown in the previous figures. The left edge of the sleeve 30, made of a contrasting colour, indicates together with the scale division a certain pressure. In order to be able to see the edge of the sleeve 30 properly, the circumference 34 of the housing has, there where the scale division has been applied, the shape of a square with rounded corners.

The rounded corners have a smaller radius of curvature than the radius of the inner wall 33, so that at these corners a magnifying glass 35 is formed.

Instead of a scale division on the outer surface of the housing it is obviously also possible to apply a scale division on the outside of the insert 37 which will be covered to a greater or lesser degree by the sleeve 30. The magnifying effect ensures that the scale division will appear to be larger.

The illustrated and described axial embodiment of the pressure gauges renders them exceptionally suitable for incorporation in a tube-like catheter section, without them being an impediment. Other embodiments of the pressure gauge are also possible however.

## Claims

1. Balloon catheter comprising a tube-like basic member with a distal and a proximal end and at least two lumens each of which is connected to a connecting piece by a single catheter section at the proximal end and wherein a balloon-shaped member is attached close to the distal end of which an internal space of which is connected to one of the lumens through which it can be filled with fluid and wherein a pressure gauge has been received in the single catheter section leading to the balloon-shaped member, said pressure gauge comprising an elongated housing with an inlet and an outlet placed axially opposite each other, wherein a piston is received, movable against a spring by the pressure to be measured, and wherein the piston and a scale division work together either directly or indirectly, for indicating the measured pressure.

2. Balloon catheter as claimed in claim 1, wherein the inside of the rectangular housing comprises a first axial cylinder bore and opposite to that a second axial cylinder bore with a smaller diameter, the piston comprises two parts working together with the cylinder bores respectively and wherein a continuous channel has been formed from the inlet through the first cylinder bore, axially through the piston, through the second cylinder bore to the outlet.

3. Balloon catheter as claimed in claim 1 or 2, wherein the housing has been manufactured of a transparent plastic material and to a wall of which a scale division has been arranged, working together with an edge of the piston.

4. Balloon catheter as claimed in claim 3, wherein at the scale division the housing has a cross-section with a polygonal shape with rounded corners.
